Europäisches Patentamt

European Patent Office

Office européen des brevets

(19)

(11) Veröffentlichungsnummer: **0 212 602**
**B1**

# EUROPÄISCHE PATENTSCHRIFT

(12)

(45) Veröffentlichungstag der Patentschrift:
21.09.88

(51) Int. Cl.⁴: **B 01 J 23/90 //**
**C07C85/11**

(21) Anmeldenummer: **86111377.7**

(22) Anmeldetag: **18.08.86**

(54) **Verfahren zur Regeneration von Katalysatoren für die Gasphasenreduktion von aromatischen Nitroverbindungen.**

(30) Priorität: **29.08.85 DE 3530820**

(43) Veröffentlichungstag der Anmeldung:
**04.03.87 Patentblatt 87/10**

(45) Bekanntmachung des Hinweises auf die Patenterteilung:
**21.09.88 Patentblatt 88/38**

(84) Benannte Vertragsstaaten:
**BE DE FR GB IT**

(56) Entgegenhaltungen:
**DE-A-2 152 539**
**US-A-2 963 443**
**US-A-3 243 383**
**US-A-3 451 942**

(73) Patentinhaber: **BAYER AG, Konzernverwaltung RP Patentabteilung, D-5090 Leverkusen 1 Bayerwerk (DE)**

(72) Erfinder: **Stammann, Günter, Dr., Silesiusstrasse 78, D-5000 Köln 80 (DE)**
Erfinder: **Kricsfalussy, Zoltan, Dr., Franz- Marc- Strasse 32, D-5090 Leverkusen 1 (DE)**
Erfinder: **Waldmann, Helmut, Dr., Henry- T.- von- Böttinger- Strasse 15, D-5090 Leverkusen 1 (DE)**
Erfinder: **Schneider, Joachim, Dr., Heyenbaumstrasse 86a, D-4150 Krefeld (DE)**
Erfinder: **Medem, Harald, Dr., Scheiblerstrasse 85, D-4150 Krefeld (DE)**

EP 0 212 602 B1

**0 212 602**

## Beschreibung

Die vorliegende Erfindung betrifft ein verbessertes Verfahren zur Regeneration von Katalysatoren für die Gasphasenreduktion von aromatischen Nitroverbindungen zu aromatischen Aminen.

Die technisch bedeutendsten Verfahren zur Herstellung aromatischer Amine sind Reduktionen der entsprechenden Nitroverbindungen in der Gasphase. Beispielsweise wird Anilin, ein Zwischenprodukt für Kautschukchemikalien und für Methylendiphenyldiisocyanat (Vorprodukt für Polyurethane), überwiegend durch Reduktion von Nitrobenzol mit Wasserstoff in der Gasphase in großen Mengen produziert. Man kann Anilin auch nach einem neueren Verfahren durch Reduktion von Nitrobenzol mit Erdgas in Gegenwart von Kupferchromit enthaltenden Katalysatoren und Wasserdampf herstellen (siehe z. B. EP-OS-0 087 690).

Bei solchen Reduktionen in der Gasphase verliert der Katalysator im Laufe der Zeit an Aktivität, d. h. der Umsatz an Nitroverbindung geht allmählich zurück, häufig verbunden mit einem Absinken der Selektivität. Die technisch verwendeten Katalysatoren sind zwar regenerierbar, doch werden dann Regenerationen in immer kürzeren Abständen nötig. Über die Regeneration und Regenerierbarkeit wird beispielsweise in Kirk-Othmer, Encyclopedia of Chemical Technology, 3. Auflage, Band 5, Seite 21 (1979) und in Ullmanns Encyclopädie der technischen Chemie, 4. Auflage, Band 13, Seite 565 (1977) berichtet. Üblich zur Regeneration von Katalysatoren zur Nitrobenzolreduktion ist das Behandeln mit Luft (siehe z. B. US-PS-2 891 094) oder mit Luft/Dampf-Gemischen (siehe z. B. US-PS-2 292 879), gefolgt von einer reduzierenden Nachbehandlung. Für das o. a. Reduktionsverfahren mittels Erdgas wird das Behandeln mit Luft/Dampf-Gemischen angegeben.

Durch Katalysatorregeneration verursachte Unterbrechungen in der Aminproduktion beeinträchtigen nicht nur die Wirtschaftlichkeit, sondern stellen auch eine schwerwiegende Störung für die Destillationsstufen bei der kontinuierlichen Aufarbeitung des Gemisches dar, das nach der Reduktion vorliegt. Ähnliche Probleme ergeben sich mit der Wasserstoffversorgung, die häufig durch Dampfreformieren kontinuierlich erfolgt. Während der Regenerationsphase muß dann der bereitgestellte, nicht verwendete Wasserstoff verbrannt oder zum Heizwert abgegeben werden, da der Reformer nicht vorübergehend abgestellt werden kann, sondern mit verminderter Kapazität (etwa 40 % der Vollast) weiterbetrieben werden muß. Die Wirtschaftlichkeit solcher Reduktionsverfahren zur Herstellung aromatischer Amine könnte deshalb verbessert werden, wenn es gelingt, die Katalysatorstandzeiten zu erhöhen.

Es wurde nun ein Verfahren zur Regeneration von Katalysatoren für die Gasphasenreduktion von aromatischen Nitroverbindungen zu aromatischen Aminen gefunden, das dadurch gekennzeichnet ist, daß man den Katalysator mit einem Gasgemisch, welches a) Sauerstoff, b) Stickstoffverbindungen in Form anorganischer und/oder organischer Amine und/oder Stickoxide und gegebenenfalls c) Wasserdampf enthält, bei erhöhter Temperatur behandelt.

Das erfindungsgemäße Regenerationsverfahren kann ganz allgemein auf Katalysatoren angewendet werden, die für die Reduktion beliebiger aromatischer Nitroverbindungen zu den entsprechenden aromatischen Aminen in der Gasphase eingesetzt werden sind. Die hergestellten Amine können dabei carbocyclische oder heterocyclische aromatische Amine sein und einschließlich der möglichen Substituenten Molekulargewichte bis zu beispielsweise 200 aufweisen. Bevorzugt wird das erfindungsgemäße Regenerationsverfahren auf Katalysatoren angewendet, die für die Herstellung von 1,3-Diaminobenzol, reinen Toluidinisomeren, Gemischen von Toluidinisomeren und/oder Anilin aus den entsprechenden Nitroverbindungen verwendet worden sind, besonders bevorzugt auf Katalysatoren, die zur Herstellung von Anilin aus Nitrobenzol verwendet worden sind.

Die Katalysatoren, auf die das erfindungsgemäße Regenerationsverfahren angewendet werden kann, können im Festbett, im Fließbett oder im Wirbelbett eingesetzt worden sein. Dementsprechend kann auch das erfindungsgemäße Regenerationsverfahren als Festbett-, Fließbett- oder Wirbelbettverfahren durchgefuhrt werden. Vorzugsweise wird das erfindungsgemäße Regenerationsverfahren auf Katalysatoren angewendet, die im Festbett eingesetzt worden sind, und es wird ebenso bevorzugt im Festbett durchgeführt. Besonders bevorzugt wird das erfindungsgemäße Regenerationsverfahren auf Festbettkatalysatoren angewendet, die in Rohrreaktoren oder in Rohrreaktorbündeln eingesetzt worden sind.

Erfindungsgemäß werden die jeweiligen Katalysatoren mit a) Sauerstoff, b) Stickstoffverbindungen in Form anorganischer und/oder organischer Amine und/oder Stickoxiden und gegebenenfalls c) Wasserdampf enthaltenden Gasgemischen in Kontakt gebracht. Dabei kann jeweils nur eine Stickstoffverbindung oder eine beliebige Mischung solcher Stickstoffverbindungen zum Einsatz gelangen.

Katalysatoren, die nach dem erfindungsgemäßen Verfahren regeneriert werden können, sind solche, die als Reduktionskatalysatoren für die Reduktion von aromatischen Nitroverbindungen zu aromatischen Aminen in der Gasphase verwendet worden sind, wobei die Reduktion z. B. mit Wasserstoff oder Erdgas durchgeführt worden ist. Die Katalysatoren können beispielsweise die Elemente Kupfer, Eisen, Nickel, Palladium und/oder Platin in metallischer Form oder in Form von Verbindungen enthalten und gegebenenfalls durch weitere Metalle oder Metallverbindungen, insbesondere durch Metalloxide, modifiziert sein. Die Metalle, z. B. Kupfer und Nickel, können auch als Mischoxide, insbesondere als Chromite vorliegen. Ein Beispiel hierfür ist Kupferchromit. Die katalytisch wirksamen Verbindungen können in den verschiedensten Formen und Korngrößen vorliegen, sie können auch auf Trägern aufgebracht sein. Beispielsweise können sie als sogenannte Schalenkontakte auf Aluminiumoxidträgern vorliegen.

Bekannte Katalysatoren für die Gasphasenreduktion von Nitroaromaten zu aromatischen Aminen, die dem erfindungsgemäßen Verfahren unterworfen werden können, sind beispielsweise beschrieben in L.F. Albright,

2

0 212 602

F.H. v. Munster, J.C. Forman, Chem. Eng., Nov. 6 (1967) Seiten 251 - 259, DE-OS-2 250 844, DE-OS-2 024 282, US-PS-2 891 094, DE-OS-2 135 155, DE-OS-2 135 154, DE-OS-2 214 056, DE-OS-2 244 401 und EP-OS-0 011 090 (entspricht US-PS-4 265 834).

In einem neueren Verfahren (siehe EP-OS-087 690) wird Anilin durch Reduktion von Nitrobenzol mit Erdgas in Gegenwart von Wasserdampf und Kupferchromit-Katalysatoren in der Gasphase hergestellt. Für dieses Verfahren ist die erfindungsgemäße Regeneration in besonderer Weise geeignet.

Bevorzugt wird das erfindungsgemäße Verfahren auf Katalysatoren angewendet, die Palladium und/oder Kupfer in metallischer Form und/oder in Form von Verbindungen enthalten. Besonders bevorzugt wird das erfindungsgemäße Regenerationsverfahren auf Katalysatoren angewendet, die in Herstellungsverfahren gemäß der EP-OS-0 011 090 und der EP-OS-0 087 690 eingesetzt worden sind.

Das erfindungsgemäß zur Katalysatorregeneration zu verwendende Gasgemisch kann z. B., pro Liter Schüttvolumen des jeweiligen Katalysators, 5 bis 100 l/h (Normliter) Sauerstoff, 2 bis 1000 g/h Stickstoffverbindung und gegebenenfalls 50 bis 2000 g/h dampfförmiges Wasser enthalten. Vorzugsweise ist Wasserdampf zugegen, bevorzugt in einer Menge von 200 bis 1000 g/h pro Liter Schüttvolumen des Katalysators.

Der Sauerstoff wird vorzugsweise verdünnt mit Inertgasen, beispielsweise Stickstoff und/oder Kohlendioxid, verwendet. Besonders bevorzugt wird Sauerstoff in Form von Luft oder Luft/Stickstoff-Gemischen eingesetzt. Der Volumenanteil an Inertgasen kann z. B. bis zum 20-fachen der Sauerstoffmenge betragen. Bevorzugt sind Inertgasmengen bis zum 10-fachen Volumenanteil der Sauerstoffmenge. Zu beachten ist ferner, daß die Sauerstoffmenge die im Katalysatorbett auftretenden Temperaturen beeinflußt.

Es ist ein wesentliches Merkmal des erfindungsgemäßen Verfahrens, daß das eingesetzte Gasgemisch Stickstoffverbindungen in Form anorganischer und/oder organischer Amine und/oder Stickoxide enthält.

Verfahren zur Reaktivierung von Katalysatoren durch Zugabe von wäßrigem Ammoniak sind bekannt (siehe beispielsweise US-PS-4 139 433, US-PS-4 107 031, US-PS-4 432 953 und DE-OS-2 459 761). Diese Verfahren sind jedoch für die Regeneration von Katalysatoren bei erhöhter Verfahrenstemperatur in der Gasphase nicht geeignet, da die Ammoniakbehandlung in flüssiger, wäßriger Phase stattfinden muß. Somit sind diese Verfahren nicht in der Gasphase anwendbar. Ferner können evtl. vorhandene koksartige Ablagerungen auf dem Katalysator nicht mit wäßrigenn Ammoniak entfernt werden.

Als anorganisches Amin kann erfindungsgemäß beispielsweise Ammoniak als solches oder, vor seiner Verdampfung, in Form beliebig konzentrierter wäßriger Lösungen eingesetzt werden. Beispielsweise können solche wäßrigen Lösungen 1 bis 40-Gew.-% Ammoniak enthalten.

Als anorganische Amine kommen weiterhin z. B. Hydrazine und/oder Hydroxylamine infrage. Bevorzugt ist Ammoniak. Als organische Amine kommen z. B. solche mit 1 bis 6 C-Atomen und 1 bis 3 N-Atomen infrage, wobei das Atomverhältnis C : N beispielsweise 2 : 1 bis 1 : 2 betragen kann. Bevorzugt ist hier Methylamin und 1,2-Ethylendiamin. Als Stickoxide kommen beispielsweise $NO$, $NO_2$, $N_2O$, $N_2O_2$, $N_2O_3$, $N_2O_4$ und $N_2O_5$ infrage, die als Einzelverbindungen oder als Gemische beliebiger Art untereinander vorliegen können. Stickoxide können gasförmig oder in Form beliebig konzentrierter, wäßriger, zu verdampfender Lösungen eingesetzt werden. Bevorzugt sind Stickoxide, in denen der Stickstoff formal die Oxidationsstufe +1, +2 oder +3 aufweist.

Es können auch solche Stickstoffverbindungen eingesetzt werden, die sich erst unter den Bedingungen des erfindungsgemäßen Verfahrens ganz oder teilweise in anorganische oder organische Amine oder Stickoxide umwandeln. Beispiele für solche Stickstoffverbindungen sind substituierte Hydrazine, N- und/oder O-substituierte Hydroxylamine, Harnstoffe, Carbonsäureamide, Nitrile und Semicarbazide, vorzugsweise jeweils solche mit bis zu 4 Kohlenstoffatomen und C : N-Atomverhältnissen von 2 : 1 bis 1 : 2. Auch Hydrate, Carbonate, Hydrogencarbonate und andere salzartige Verbindungen von anorganischen und organischen Aminen können in das erfindungsgemäße Regenerationsverfahren eingesetzt werden.

Besonders bevorzugt werden als Stickstoffverbindungen Ammoniak und 1,2-Ethylendiamin eingesetzt, ganz besonders bevorzugt Ammoniak.

Vorzugsweise wird die Komponente b) des Gasgemisches in einer Menge von 5 bis 200 g/h pro Liter Schüttvolumen des jeweiligen Katalysators verwendet.

Die einzelnen Komponenten des erfindungsgemäß anzuwendenden Gasgemisches können dem zu regenerierenden Katalysator getrennt oder gemeinsam zugeführt werden. Sofern als Komponente b) Ammoniak oder ein organisches Amin verwendet wird ist es im allgemeinen vorteilhaft diese Komponente zusammen mit Wasser einzusetzen und z. B. eine wäßrige Ammoniak- oder Aminlösung gemeinsam zu verdampfen. Solche wäßrigen Lösungen können, soweit entsprechende Löslichkeiten vorhanden sind, z. B. 1 bis 50-Gew.-% Ammoniak oder organisches Amin enthalten. Vorzugsweise weisen solche Lösungen mindestens 3-Gew.-% und höchstens der Sättigung entsprechende Konzentrationen auf. Auf diese Weise kann auch Wasser dem erfindungsgemäßen Verfahren zugefügt werden, das dann den gegebenenfalls einzusetzenden Wasserdampf ganz oder teilweise, vorzugsweise ganz, liefert.

Das erfindungsgemäße Verfahren kann in Abwesenheit oder in Anwesenheit von Wasserdampf durchgeführt werden. Vorzugsweise wird in Gegenwart von Wasserdampf gearbeitet, da dann im allgemeinen günstigere Ergebnisse erzielt werden und zur Beachtung von Explosionsgrenzen dann weniger Aufwand entsteht.

Das erfindungsgemäße Verfahren zur Katalysatorregeneration kann extern, d. h. mit ausgebautem Katalysator in einer gesonderten Apparatur durchgeführt werden. Wirtschaftlich vorteilhafter, insbesondere hinsichtlich der Zeit- und Arbeitsersparnis, ist im allgemeinen die ebenfalls mögliche in-situ-Regeneration des Katalysators. Der Katalysator wird dabei im Reaktor für die Aminproduktion ohne Ausbau des Katalysators regeneriert.

Die erfindungsgemäße Katalysatorregeneration findet bei erhöhter Temperatur statt. Diese kann z. B. im Bereich von 200 bis 600°C liegen. Vorzugsweise wird die Regenerationstemperatur dem zugrundeliegenden Prozeß der Aminproduktion angepaßt, und zwar in der Weise, daß man die Regeneration bei der gleichen Temperatur wie die Aminproduktion oder bis zu 80°C darüber, besonders bevorzugt bei bis zu 50°C darüber, durchführt.

In einer speziellen Ausführungsform kann das erfindungsgemäße Verfahren wie folgt durchgeführt werden:

Unter Beibehaltung der Katalysatortemperatur wird die Zufuhr des Nitroaromaten für die Aminherstellung unterbrochen und das Reduktionsmittel durch einen Stickstoffstrom ersetzt. Dann wird beispielsweise 5-%-iges Ammoniakwasser zugespeist und verdampft. Unter allmählicher Verminderung des Stickstoffstromes wird entsprechend Luft in solchem Maße zugespeist, daß sich die Katalysatortemperatur maximal 50°C über der Temperatur der Aminproduktion einstellt. Wenn in der Katalysatorzone keine Temperaturänderung mehr zu beobachten oder im Abgas kein $CO_2$ mehr nachweisbar ist, wird die Regeneration, gegebenenfalls nach weiteren 0,1 bis 5 Stunden, beendet, z. B. indem man den Luftstrom wieder durch einen Stickstoffstrom ersetzt, die Zuspeisung von Ammoniaklösung beendet und nach genügender Zwischenspülung mit Stickstoff wieder das Reduktionsgas, z. B. Wasserstoff oder Erdgas, und den Nitroaromaten in die Aminproduktion einspeist.

Im allgemeinen ist es nicht notwendig, den regenerierten Katalysator gesondert reduzierend nachzubehandeln. Dies gilt insbesondere dann, wenn die aromatische Nitroverbindung bei vollem Einsatz des Reduktionsgases für die Aminproduktion erst im Verlauf von beispielsweise 0,5 bis 8 Stunden schrittweise auf die gewünschte Sollmenge gebracht wird.

Im allgemeinen kann das erfindungsgemäße Regenerationsverfahren bei Normaldruck oder dem zur Überwindung des Druckverlustes der Katalysatorschüttung erforderlichen leichten Überdruck oder der zur Aufrechterhaltung eines Fließ- oder Wirbelbettes benötigten Druckdifferenz, durchgeführt werden. Ein Arbeiten bei Unterdruck, beispielsweise bei bis zu 0,1 bar, oder bei Überdruck, beispielsweise bei bis zu 30 bar, ist jedoch ebenso möglich. Dies kann dann vorteilhaft sein, wenn das Verfahren der Aminproduktion nicht bei Normaldruck betrieben wird und das Regenerationsverfahren aus technischen Gründen beim gleichen Druck durchgeführt werden soll wie die Aminproduktion.

In einer abgewandelten Form des erfindungsgemäßen Regenerationsverfahrens kann dieses auch an frischen Katalysatoren als Präformierverfahren durchgeführt werden. Das Vorgehen entspricht dann dem oben beschriebenen, jedoch ist die Behandlung mit dem Regenerationsgas, das die Komponenten a) und b) und gegebenenfalls c) enthält, im allgemeinen kürzer, sie sollte jedoch im allgemeinen mindestens 3 Stunden dauern.

Die Vorteile des erfindungsgemäßen Regenerationsverfahrens sind zum einen die Möglichkeit, in situ regenerieren zu können und zum anderen die deutliche Verlängerung der Zyklen zwischen einzelnen Regenerationen. Es ist besonders überraschend, daß diese für die Aminproduktion wirtschaftlich bedeutenden Vorteile durch die sehr einfachen, technisch leicht zu realisierenden Maßnahmen des erfindungsgemäßen Regenerationsverfahrens erzielt werden können.

Die nachstehenden Beispiele illustrieren das erfindungsgemäße Verfahren, ohne es in irgendeiner Weise auf diese Ausführungsformen zu beschränken.

## Beispiele

Verwendete Katalysatoren:

Katalysator A:
Dieser Katalysator wurde entsprechend Beispiel 1 der DE-OS-2 849 002 hergestellt und enthielt 6 g Palladium, 9 g Vanadium und 6 g Blei pro Liter Aluminiumoxidträger. Dieser Katalysator wurde in den Beispielen 1, 2 und 11 verwendet.

Katalysator B:
Käuflicher Kupferchromit-Katalysator (Ventron 11845, Alfa-Katalog 1984, Europäische Ausgabe, Seite 165). Nach Katalogangabe ist der Katalysator charakterisiert durch einen Gehalt von 42-Gew.-% CuO und 38-Gew.-% $Cr_2O_3$. Eine Debye-Scherrer-Röntgenaufnahme zeigte, daß der Katalysator überwiegend aus Kupfer-(II)-chromit ($CuCr_2O_4$) besteht. Dieser Katalysator wurde in den Beispielen 3 bis 10 verwendet.

Erläuterungen zu den Beispielen 1, 2 und 11:
Die Beispiele 1, 2 und 11 wurden in einem als Differential-Hydrier-Reaktor betriebenen Käfigreaktor von 300 ml Gesamtvolumen bei Normaldruck durchgeführt. Derartige Reaktoren sind besonders für kinetische Untersuchungen geeignet und detailliert beschrieben z. B. von A. Schulz-Walz, D.C. Hempel, Z. Kricsfalussy und C. Rasp in Chem. Ing. Technik, 53, (8), 637 - 640 (1981). Der Reaktor wurde mit 50 ml Katalysator A beschickt. Die Flüssigkeiten wurden mit einer Minidosierpumpe zugespeist, vor dem Reaktor in einem Vorheizer bei 300°C verdampft und vereint mit den Einsatzgasströmen dem Reaktor zugeführt. Der gasförmige Ausgangsstrom wurde nacheinander durch eine mit Eis und eine mit festem $CO_2$ gekühlte Kühlfalle geleitet, um die kondensierbaren Reaktionsprodukte abzuscheiden. Die Kondensate wurden im Abstand von 8 Stunden vereint und gaschromatographisch quantitativ analysiert.

Die im Vergleich zu den Beispielen der DE-OS-2 849 002 erhöhte Reaktionstemperatur ist beabsichtigt, um die Katalysatordesaktivierung, die sich normalerweise über einen Zeitraum von 900 bis 1500 Stunden erstreckt, mit Zeitraffereffekt untersuchen zu können. Der erhöhten Reaktionstemperatur wegen liegen auch die Anilinselektivitäten dieser Beispiele niedriger, als unter optimalen Reaktionsbedingungen für die Anilinherstellung möglich wäre.

**Beispiel 1** (zum Vergleich)

Einsätze:
50 ml frischer Katalysator A
20 ml/h Nitrobenzol (0,195 mol/h)
25 l/h Wasserstoff (1,040 mol/h)
1,5 l/h Stickstoff (0,062 mol/h)

Reaktionsbedingungen:
Katalysatortemperatur: 420°C
Druck: 1 bar

Nach 8 Stunden Reaktionszeit betrug der Umsatz an Nitrobenzol 91 % und die Selektivität der Anilinbildung, bezogen auf umgesetztes Nitrobenzol, 92 %. Im Verlaufe von 200 Stunden ging der Umsatz an Nitrobenzol auf 61 % zurück und die Selektivität der Anilinbildung, bezogen auf umgesetztes Nitrobenzol, auf 66 %.

Nach 200 Betriebsstunden wurde der Katalysator wie folgt regeneriert:

Der Nitrobenzoleinsatz und der Wasserstoffeinsatz wurden gestoppt und über den bei 300°C betriebenen Vorerhitzer wurden 12,5 g/h Wasserdampf erzeugt und zusammen mit 16 l/h Stickstoff in den Reaktor eingeleitet. Es wurde schrittweise Luft zudosiert und entsprechend der Stickstoffeinsatz zurückgenommen bis ein Wert von 8 l/h Stickstoff und 8 l/h Luft erreicht war. Das Zudosieren von Luft erfolgt unter Kontrolle der Katalysatortemperatur, so daß 450°C nicht überschritten wurden. Mit den Luft/Stickstoff/Wasserdampf-Gemisch wurde solange regeneriert bis im Abgasstrom IR-spektroskopisch kein $CO_2$ mehr nachweisbar war, was 13 Stunden dauerte.

Danach wurde mit Stickstoff zwischengespült, der Wassereinsatz gestoppt, die ursprüngliche Katalysatortemperatur und der $N_2/H_2$-Strom wieder eingestellt. Der Nitrobenzoleinsatz wurde innerhalb von 30 Minuten auf die ursprüngliche Sollmenge gebracht und so die Anilinherstellung wieder aufgenommen. Nach weiteren 8 Stunden betrug der Umsatz an Nitrobenzol 92 % und die Selektivität der Anilinbildung, bezogen auf umgesetztes Nitrobenzol, 89 %.

Im Verlauf von weiteren 72 Stunden ging der Umsatz an Nitrobenzol auf 37 % zurück und die Selektivität der Anilinbildung, bezogen auf umgesetztes Nitrobenzol, auf 17 %.

**Beispiel 2** (erfindungsgemäß)

Es wurde mit frischem Katalysator A wie in Beispiel 1 verfahren. Nach 8 Stunden Reaktionszeit betrug der Umsatz an Nitrobenzol 91 % und die Selektivität der Anilinbildung, bezogen auf umgesetztes Nitrobenzol, ebenfalls 91 %. Im Verlaufe von 200 Stunden ging der Umsatz an Nitrobenzol auf 62 % zurück und die Selektivität der Anilinbildung auf 68 %.

Danach wurde der Katalysator bei Bedingungen wie in Beispiel 1 angegeben regeneriert, jedoch wurden anstelle von 12,5 g/h Wasserdampf 12,5 g/h einer wäßrigen, 5-%-igen Ammoniaklösung eingesetzt.

Anschließend wurde die Herstellung von Anilin fortgeführt wie in Beispiel 1 beschrieben.

8 Stunden nach Beendigung der Regeneration (= nach dem Beginn des erneuten Einspeisens der Sollmenge an Nitrobenzol) betrug der Umsatz an Nitrobenzol 90 % und die Selektivität der Anilinbildung, bezogen auf umgesetztes Nitrobenzol, 88 %. Im Verlaufe von weiteren 200 Stunden änderten sich diese Werte nur unwesentlich. Der Umsatz an Nitrobenzol betrug dann 88 %, die Selektivität der Anilinbildung, bezogen auf umgesetztes Nitrobenzol, 90 %.

**Beispiele 3 bis 10**

Die Beispiele 3 bis 6 und das Beispiel 8 sind Vergleichsbeispiele, die Beispiele 7, 9 und 10 sind erfindungsgemäße Beispiele, die den günstigen Einfluß des erfindungsgemäßen Verfahrens auf die Anilinherstellung aus Nitrogemäßen Verfahrens auf die Anilinherstellung aus Nitrobenzol mit Erdgas und Wasserdampf an einem Kupferchromit-Katalysator gemäß. EP-OS-0 087 690 belegen.

Der verwendete Rohrreaktor entsprach denn Reaktor von Beispiel 15 der EP-OS-0 087 690. Abweichend von dem dort beschriebenen Verfahren wurde die Anilinherstellung bei Überdruck und mit Kreisgas durchgeführt, d. h. die gasförmigen, durch Kühlwasser nicht kondensierten Komponenten wurden mittels eines Kreisgasgebläses zum Reaktoreingang zuruckgeführt. Die Einsatzmengen und Reaktionsbedingungen der Anilinherstellung sind in Fußnote 1 zur Tabelle 1 angegeben.

Die Regenerationen wurden wie folgt durchgeführt:

Das Vorgehen entsprach Beispiel 1. Die Einsatzmengen waren entsprechend dem größeren Katalysatorschüttvolumen folgende:

Zu Beginn der Regeneration: 350 l/h Stickstoff.

Während der Regeneration: 175 l/h Stickstoff und 175 l/h Luft und

550 g/h Wasser (bei den Beispielen 3 bis 6 und 8), 550 g/h 5-%-ige wäßrige Ammoniaklösung (bei den Beispielen 7 und 9) und 550 g/h 5-%-ige wäßrige Lösung von 1,2-Ethylendiamin (bei Beispiel 10).

Die Ergebnisse der Beispiele 3 bis 10 sind in Tabelle 1 zusammengefaßt. Die Selektivität der Anilinbildung lag bei diesen Beispielen bei 95 $\pm$ 1-mol-%, bezogen auf umgesetztes Nitrobenzol. Der Umsatzabfall über die Zyklusdauer ist annähernd linear. Zu Beginn des Beispiels 6 wies der Kupferchromit-Katalysator 7500 Betriebsstunden für die Anilinproduktion entsprechend der EP-OS-0 087 690 auf. Zu diesem Zeitpunkt war die Zyklusdauer bereits von ursprünglich ca. 300 Stunden auf 168 Stunden gefallen. Die erfindungsgemäßen Beispiele 7, 9 und 10 zeigen, daß durch die erfindungsgemäße Regeneration wieder eine Zyklusdauer in der ursprünglichen Größenordnung erreicht werden kann.

**Tabelle 1**

| Beispiel Nr.[1] | Produktionszyklus Nr. | Nitrobenzolumsatz[2] [%] | | Zyklusdauer [Std.] | Regeneration <u>vor</u> dem Zyklus |
|---|---|---|---|---|---|
| | | Beginn | Ende | | |
| 3 | 1 | 96 | 62 | 240 | ohne $NH_3$ |
| 4 | 2 | 93 | 66 | 264 | ohne $NH_3$ |
| 5 | 3 | 95 | 65 | 312 | ohne $NH_3$ |
| 6 | 40 | 92 | 50 | 168 | ohne $NH_3$ |
| 7 | 41 | 95 | 67 | 304 | mit $NH_3$ |
| 8 | 42 | 95 | 63 | 210 | ohne $NH_3$ |
| 9 | 43 | 96 | 65 | 304 | mit $NH_3$ |
| 10 | 44 | 95 | 65 | 304 | mit $H_2N\text{-}CH_2CH_2\text{-}NH_2$ |

1) Einsatzmengen:        550 g/h Nitrobenzol
                                 550 g/h Wasser
                                 260 l/h Erdgas (Normliter)

Reaktionsbedingungen:    4 bar Druck
                                 420° C maximale Katalysatortemperatur
                                 300 l/h Kreisgas (Effektivliter)

2) Nitrobenzolumsatz zu Beginn und zu Ende des jeweiligen Produktionszyklus.

**Beispiel 11** (erfindungsgemäß)

Es wurde mit frischem Katalysator A wie in Beispiel 1 beschrieben verfahren. Nach 8 Stunden Reaktionszeit betrug der Umsatz an Nitrobenzol 91,5 % und die Selektivität der Anilinbildung, bezogen auf umgesetztes Nitrobenzol 91 %. Im Verlaufe von 200 Stunden ging der Umsatz von Nitrobenzol auf 63 % zurück und die Selektivität der Anilinbildung auf 66 %.

Danach wurde wie in Beispiel 1 angegeben regeneriert, jedoch wurde dem Regeneriergas 1,1g/h Stickstoffmonoxid (NO) zugefügt. Anschließend wurde die Herstellung von Anilin fortgeführt wie in Beispiel 1 beschrieben.

8 Stunden nach Beendigung der Regeneration (= nach dem Beginn des erneuten Einspeisens der Sollmenge an Nitrobenzol) betrug der Umsatz an Nitrobenzol 91 % und die Selektivität der Anilinbildung, bezogen auf umgesetztes Nitrobenzol 92 %. Im Verlauf von weiteren 144 Stunden ging der Umsatz an Nitrobenzol auf 56 % zurück und die Selektivität der Anilinbildung, bezogen auf umgesetztes Nitrobenzol, auf 65 %.

**Beispiel 12** (erfindungsgemäß)

Ein Einrohr-Hydrierreaktor (Rohrdurchmesser 32 mm, Rohrlänge 1300 mm) wurde mit einem Liter eines Katalysators gefüllt, der 9 g Palladium, 9 g Vanadium und 3 g Blei pro Liter Aluminiumoxidträger enthielt (hergestellt entsprechend Katalysator A, s. Einleitung des Beispielteils). Die Reaktionstemperatur wurde durch ein siedendes Wärmeträgeröl bei 260 bis 280°C gehalten. Im Wasseratoffstrom wurde Nitrobenzol bei 160 bis 180°C verdampft und von unten nach oben durch den Reaktor geleitet. Die Belastung betrug 0,6 kg Nitrobenzol/h·l Kontakt bei 6 Mol $H_2$ pro Mol Nitrobenzol. Das gasförmige Reaktionsprodukt wurde kondensiert und gaschromatographisch untersucht. Der Nitrobenzolumsatz lag bei 100 %, die Anilinselektivität bei 100 - 99,5 %. Nach dem Abfall des Nitrobenzolumsatzes auf 99,5 % wurde der Versuch abgebrochen. Die Standzeit betrug 1690 Stunden. Die anschließende Regeneration wurde so durchgeführt, daß gleichzeitig Stickstoff, Luft und wäßrige Ammoniaklösung über den Nitrobenzolverdampfer eingeleitet wurden, bis im Austritt kein $CO_2$ mehr nachweisbar war.

Einsatzmengen:  200 l Stickstoff pro Stunde
200 l Luft pro Stunde
200 ml 25-%-ige wäßrige Ammoniaklösung pro Stunde

Regenerationsdauer: 4 Stunden

Nach dieser Regeneration wurde der Katalysator erneut zur Hydrierung von Nitrobenzol eingesetzt. Die Versuchsbedingungen waren die gleichen wie oben beschrieben.
Der Nitrobenzolumsatz betrug wiederum 100 %, die Anilinselektivität betrug wiederum 99,0 - 99,5 %. Dieser Produktionszyklus erreichte eine Standzeit (= Abfall des Nitrobenzolumsatzes auf 99,5 %) von 2940 Stunden.

**Beispiel 13** (zum Vergleich)

Es wurde ebenso verfahren wie in Beispiel 12, jedoch wurde nach 1650 Stunden die Regeneration ohne Zusatz einer wäßrigen Ammoniaklösung ausgeführt. Im darauffolgenden Produktionszyklus erreichte der Katalysator eine Standzeit von 1820 Stunden (bis zum Abfall des Nitrobenzolumsatzes auf 99,5 %).

**Beispiel 14** (erfindungsgemäß)

Es wurde verfahren wie in Beispiel 12, jedoch wurde anstelle von Nitrobenzol eine entsprechende Menge o-Nitrotoluol eingeleitet, das zu o-Toluidin reduziert wurde. Die Umsätze und Selektivitäten waren anfänglich wie in Beispiel 12 angegeben. Nach 520 Stunden war der o-Nitrotoluol-Umsatz auf 99,5 % zurückgegangen. Es wurde regeneriert wie in Beispiel 12 angegeben. Nach der Regeneration dauerte es 795 Stunden, bis der o-Nitrotoluol-Umsatz wieder auf 99,5 % gefallen war.

**Beispiel 15** (zum Vergleich)

Es wurde verfahren wie in Beispiel 14, jedoch wurde nach 550 Stunden die Regeneration ohne Zusatz einer wäßrigen Ammoniaklösung durchgeführt. Im darauffolgenden Produktionszyklus erreichte der Katalysator nur eine Standzeit von 515 Stunden (bis zum Abfall des o-Nitrotoluol-Umsatzes auf 99,5 %).

**Beispiel 16** (erfindungsgemäß)

Es wurde verfahren wie in den Beispielen 12 und 14, jedoch wurde eine entsprechende Menge p-Nitrotoluol eingeleitet, das zu p-Toluidin reduziert wurde. Die Umsätze und Selektivitäten waren anfänglich wie im Beispiel 12 angegeben. Nach 730 Stunden war der p-Nitrotoluol-Umsatz auf 99,5 % zurückgegangen. Es wurde regeneriert wie in Beispiel 12 angegeben. Nach der Regeneration dauerte es 1045 Stunden, bis der p-Nitrotoluol-Umsatz wieder auf 99,5 % gefallen war.

**Beispiel 17** (zum Vergleich)

Es wurde verfahren wie in Beispiel 16, jedoch wurde nach 690 Stunden die Regeneration ohne Zusatz einer wäßrigen Ammoniaklösung durchgeführt. Im darauffolgenden Reaktionszyklus erreichte der Katalysator nur eine Standzeit von 715 Stunden (bis zum Abfall des p-Nitrotoluol-Umsatzes auf 99,5 %).

**Beispiel 18** (erfindungsgemäß)

Es wurde verfahren wie in den Beispielen 12 und 14, jedoch wurde eine entsprechende Menge m-Nitrotoluol eingeleitet, das zu m-Toluidin reduziert wurde. Die Umsätze und Selektivitäten waren anfänglich wie im Beispiel 12 angegeben. Nach 605 Stunden war der m-Nitrotoluol-Umsatz auf 99,5 % zurückgegangen. Es wurde regeneriert wie in Beispiel 12 angegeben. Nach der Regeneration dauerte es 940 Stunden, bis der m-Nitrotoluol-Umsatz wieder auf 99,5 % gefallen war.

**Beispiel 19** (zum Vergleich)

Es wurde verfahren wie in Beispiel 18, jedoch wurde nach 690 Stunden die Regeneration ohne Zusatz einer wäßrigen Ammoniaklösung durchgeführt. Im darauffolgenden Produktionszyklus erreichte der Katalysator nur eine Standzeit von 630 Stunden (bis zum Abfall des m-Nitrotoluol-Umsatzes auf 99,5 %).

**Patentansprüche**

1. Verfahren zur Regeneration von Katalysatoren für die Gasphasenreduktion von aromatischen Nitroverbindungen zu aromatischen Aminen, dadurch gekennzeichnet, daß man den Katalysator mit einem Gasgemisch, welches a) Sauerstoff und b) Stickstoffverbindungen in Form anorganischer oder organischer Amine und/oder Stickoxiden und gegebenenfalls c) Wasserdampf enthält, bei erhöhter Temperatur behandelt.

2. Verfahren nach Anspruch 1, dadurch gekennzeichnet, daß man es auf Katalysatoren anwendet, die zur Herstellung von Anilin aus Nitrobenzol verwendet worden sind.

3. Verfahren nach Ansprüchen 1 und 2, dadurch gekennzeichnet, daß die Katalysatoren die Elemente Kupfer, Eisen, Nickel, Palladium und/oder Platin in metallischer Form oder in Form von Verbindungen enthalten und gegebenenfalls durch weitere Metalle oder Metallverbindungen modifiziert sind.

4. Verfahren nach Ansprüchen 1 bis 3, dadurch gekennzeichnet, daß das Gasgemisch pro Liter Schüttvolumen des jeweiligen Katalysators 5 bis 100 l/h Sauerstoff, 2 bis 1000 g/h Stickstoffverbindung und gegebenenfalls 50 bis 2000 g/h Wasserdampf enthält.

5. Verfahren nach Ansprüchen 1 bis 4, dadurch gekennzeichnet, daß das Gasgemisch 200 bis 1000 g/h Wasser pro Liter Schüttvolumen des jeweiligen Katalysators enthält.

6. Verfahren nach Ansprüchen 1 bis 5, dadurch gekennzeichnet, daß als Stickstoffverbindungen Ammoniak, Hydrazine, Hydroxylamine, organische Amine mit 1 bis 6 C-Atomen und 1 bis 3 N-Atomen, NO, $NO_2$, $N_2O$, $N_2O_2$, $N_2O_3$, $N_2O_4$ und/oder $N_2O_5$ eingesetzt werden.

7. Verfahren nach Anspruch 6, dadurch gekennzeichnet, daß als Stickstoffverbindung Ammoniak und/oder 1,2-Ethylendiamin eingesetzt werden.

8. Verfahren nach Ansprüchen 1 bis 7, dadurch gekennzeichnet, daß die Behandlung mit dem Gasgemisch bei 200 bis 600°C durchgeführt wird.

9. Verfahren nach Anspruch 8, dadurch gekennzeichnet, daß die Behandlung mit dem Gasgemisch bei der gleichen Temperatur wie die zugrundeliegende Aminherstellung oder bis zu 80°C darüber durchgeführt wird.

10. Verfahren nach Ansprüchen 1 bis 9, dadurch gekennzeichnet, daß der Sauerstoff verdünnt mit Inertgasen verwendet wird.

**Claims**

1. Process for the regeneration of catalysts for the gas-phase reduction of aromatic nitro compounds to aromatic amines, characterized in that the catalyst is treated, at elevated temperature, with a gas mixture which contains a) oxygen, b) nitrogen compounds in the form of inorganic or organic amines and/or nitrogen oxides and, where appropriate, c) steam.

2. Process according to Claim 1, characterized in that it is employed for catalysts which have been used for the preparation of aniline from nitrobenzene.

3. Process according to Claims 1 and 2, characterized in that the catalysts contain the elements copper, iron, nickel, palladium and/or platinum in metallic form or in the form of compounds and have, where appropriate, been modified by other metals or metal compounds.

4. Process according to Claims 1 to 3, characterized in that the gas mixture contains 5 to 100 l/h oxygen, 2 to 1,000 g/h nitrogen compound and, where appropriate, 50 to 2,000 g/h steam per litre of bulk volume of the particular catalyst.

5. Process according to Claims 1 to 4, characterized in that the gas mixture contains 200 to 1,000 g/h water per litre of bulk volume of the particular catalyst.

6. Process according to Claims 1 to 5, characterized in that ammonia, hydrazines, hydroxylamines, organic amines having 1 to 6 C atoms and 1 to 3 N atoms, NO, $NO_2$, $N_2O$, $N_2O_2$, $N_2O_3$, $N_2O_4$ and/or $N_2O_5$ are used as nitrogen compounds.

7. Process according to Claim 6, characterized in that ammonia and/or 1,2-ethylenediamine are used as nitrogen compound.

8. Process according to Claims 1 to 7, characterized in that the treatment with the gas mixture is carried out at 200 to 600°C.

9. Process according to Claim 8, characterized in that the treatment with the gas mixture is carried out at the same temperature as the fundamental amine preparation or up to 80°C above that.

10. Process according to Claims 1 to 9, characterized in that the oxygen is diluted with inert gases for use.


## Revendications

1. Procédé de régénération de catalyseurs pour la réduction en phase gazeuse de nitrocomposés aromatiques, caractérisé en ce qu'on traite le catalyseur avec un mélange gazeux qui contient a) del'oxygène et b) des composés azotés sous forme d'amines minérales ou organiques et/ou d'oxydes d'azote et éventuellement de c) de la vapeur d'eau, à température élevée.

2. Procédé suivant la revendication 1, caractérisé en ce qu'on l'applique à des catalyseurs qui ont été utilisés pour la fabrication d'aniline à partir de nitrobenzène.

3. Procédé suivant les revendications 1 et 2 caractérisé en ce que les catalyseurs contiennent les éléments cuivre, fer, nickel, palladium et/ou platine sous la forme métallique ou sous la forme de composés, et en ce qu'éventuellement ils sont modifiés par d'autres métaux ou composés métalliques.

4. Procédé suivant les revendications 1 à 3 caractérisé en ce que le mélange gazeux contient par 1 de volume en vrac du catalyseur considéré, 5 à 100 l/h d'oxygène, 2 à 1000 g/h de composé azoté et éventuellement 50 à 2000 g/h de vapeur d'eau.

5. Procédé selon les revendications 1 à 4 caractérisé en ce que le mélange gazeux contient 200 à 1000 g/h d'eau par 1 de volume en vrac du catalyseur considéré.

6. Procédé selon les revendications 1 à 5, caractérisé en ce qu'on utilise comme composés azotés de l'ammoniac, des hydrazines, des hydroxylamines, des amines organiques ayant 1 à 6 atomes de carbone et 1 à 3 atomes N, NO, $NO_2$, $N_2O$, $N_2O_2$, $N_2O_3$, $N_2O_4$ et/ou $N_2O_5$.

7. Procédé selon la revendication 6, caractérisé en ce qu'on utilise comme composé azoté de l'ammoniac et/ou de la 1,2-éthylène diamine.

8. Procédé selon les revendications 1 à 7, caractérisé en ce qu'on exécute le traitement avec le mélange gazeux à 200 - 600°C.

9. Procédé selon la revendication 8, caractérisé en ce qu'on exécute le traitement avec le mélange gazeux à la même température que pour la fabrication d'amine de base ou jusqu'à 80°C au-dessus de celle-ci.

10. Procédé selon les revendications 1 à 9, caractérisé en ce qu'on utilise l'oxygène à l'état dilué avec des gaz inertes.